# EUROPEAN PATENT SPECIFICATION

(11) **EP 2 170 285 B1**
(45) Date of publication and mention of the grant of the patent: **01.10.2014**
(21) Application number: 08788908.5
(22) Date of filing: 23.07.2008
(51) Int. Cl.: A61K 9/14, A61K 9/20, A61K 31/55

(54) **PROCESS FOR THE PREPARATION OF CONTROLLED-RELEASE SOLID FORMULATIONS CONTAINING OXCARBAZEPINE, AND FORMULATIONS OBTAINABLE BY SAID PROCESS**
VERFAHREN ZUR HERSTELLUNG VON OXCARBAZEPINHALTIGEN FESTSTOFFFORMULIERUNGEN MIT GESTEUERTER FREISETZUNG UND IN DIESEM VERFAHREN HERSTELLBARE FORMULIERUNGEN
PROCÉDÉ DE PRÉPARATION DE COMPOSITIONS SOLIDES À LIBÉRATION CONTRÔLÉE CONTENANT DE L'OXCARBAZÉPINE, ET COMPOSITIONS OBTENUES PAR CE PROCÉDÉ

(30) Priority: 25.07.2007 IT MI20071502
(43) Date of publication of application: 07.04.2010
(73) Proprietor: Prime European Therapeuticals S.p.A. in forma abbreviata Euticals S.p.A., 20122 Milano (IT)
(72) Inventor: SANSO', Giovanni, I-21040 Origgio (IT); BANFI, Aldo, I-21040 Origgio (IT); BERTOLINI, Giorgio, I-21040 Origgio (IT); SILVA, Claudio, I-21040 Origgio (IT)
(74) Representative: Minoja, Fabrizio
(86) International application number: PCT/IB2008/001904
(87) International publication number: WO 2009/013594

(56) References cited:
- WO-A-02/094774
- WO-A-03/043602
- WO-A-2007/029093
- WO-A-2007/141806
- WO-A-2008/092046

## Description

### FIELD OF INVENTION

The present invention relates to a process for the preparation of controlled-release solid oral pharmaceutical formulations of oxcarbazepine, and the pharmaceutical formulations obtainable by said process.

### Introduction

The usual pharmaceutical forms that immediately release the drug have not only the advantage of immediate pharmacological action, but also some drawbacks:
- variable degrees of fluctuation of the blood levels in the same individual, with the possibility of a transient therapeutic overdose, when the plasma concentration of the active ingredient exceeds the toxicity level, followed by a period of underdosage, during which the plasma concentration of the active ingredient falls below the minimum concentration required to give the pharmacological effect;
- frequency of administration exceeding once a day, entailing a higher risk that the patient will not comply with the dosage, thus adversely affecting the efficacy of the treatment.

Partly in order to overcome these drawbacks, pharmaceutical forms with delayed release of the drug have been developed, which delay the time taken to reach the plasma levels of active ingredient needed to achieve the desired pharmacological effect. However, if the interval between administration and the time when active plasma levels are reached is too long, these forms cannot be used to obtain an immediate effect.

Consequently, to obtain an immediate pharmacological effect without the drawbacks mentioned above, and at the same time to maintain a lasting pharmacological action, a combination of the characteristics of the two forms of release described above must be used, to provide at the same time:
- rapid release of the drug, in order to obtain immediately the active blood concentration required to produce the pharmacological effect;
- a satisfactory reduction of the fluctuations in the blood levels of the active ingredient;
- a blood concentration which is active for a long enough period of time to cover the whole day with a single dose, thus allowing a reduction in frequency of administration and facilitating full compliance by the patient.

The main factors which allow the release of drugs from solid forms to be regulated are:
- the technological characteristics of the solid form, such as hardness, size, shape, etc. in the case of tablets;
- the particle-size profile of the active ingredient, i.e. not only the particle-size distribution range of the crystals of the active ingredient, but also the corresponding amounts;
- the crystalline form;
- the composition in terms of excipients, and dissolution accelerators or retardants, in the various percentages;
- the coating of the granules or tablets.

A crucial factor to ensure constant bioavailability between pharmaceutical units of the same manufacturing batch and between different batches is uniform distribution of the excipients or coating, i.e. the mixing or encapsulation technique used, as well as the manufacturing technology.

As these excipients are usually present in small percentages compared with the active ingredient, and it is therefore not always easy to achieve perfectly uniform distribution, this factor is very important.

### Prior art

A number of patents relating to controlled-release formulations have endeavoured to achieve the objectives described above.

The solid oral formulations of the specialties currently on the market containing oxcarbazepine must be taken during or after meals, with liquid, to facilitate absorption in the stomach, according to the instructions on the labels and in the information leaflets. These formulations provide an immediate, complete release and a high dose, ranging between 300 and 600 mg per dose unit. Side effects such as nausea and vomiting can be avoided by not taking the drug on an empty stomach.

Controlled-release formulations containing oxcarbazepine have recently been patented which, while maintaining an immediate, complete release, can also be taken on an empty stomach.

If the ratio between the AUCs of patients with a full and empty stomach are compared, these values fall into an interval of between 0.8 and 1.25 after a single dose.

Similarly, if the ratio between the Cₘₐₓ values of patients with a full and empty stomach are compared, it falls into an interval of between 0.7 and 1.43 after a single dose.

These formulations are characterised by an oxcarbazepine content of between 300 and 600 mg per dose unit, once-daily administration, and an *in vitro* dissolution rate of at least 90% after 30 minutes.

The solid oral forms of oxcarbazepine most recently patented consist of mixtures with a wide variety of compositions, all obtained by mixing the ingredients in the solid state; tablets, coated in various ways, which release the antiepileptic drug are obtained from said forms by direct compression or granulation and subsequent pressing in a tablet press.

Thus WO 95/29665 provides a solid oral pharmaceutical form for the treatment of epilepsy, in tablet form, wherein the release of the drug is controlled on the basis of the laws of osmosis, diffusion, bioerosion and ion exchange, with a specific bioavailability. The tablets are made by mixing and granulating the active ingredient with suitable excipients, and then coating the tablets obtained.

WO 98/35681 provides a controlled-release form of oxcarbazepine wherein the active ingredient is micronised, mixed with suitable excipients, granulated dry or wet, and compressed. The tablets are then film-coated to obtain the controlled release of the drug.

WO 01/32183 discloses a tablet form wherein the active ingredient is released in a very short time (90% in 30 minutes *in vitro)* to prevent gastric disorders. Once again, the aim is achieved by mixing the active ingredient in the solid state with suitable excipients, and giving the tablet obtained a particular coating. In practice, however, this formulation has the side effect of vomiting when administered to patients with a full stomach or who have recently eaten.

US005472714A discloses a process of obtaining double-layer tablets wherein the formation of a product of breakdown is prevented.

WO 2007/029093 A discloses a method of dispersing oxcarbazepine in a solution comprising a polymer dispersion, which is then granulated.

### Description of the invention

The present invention relates to a process for the preparation of controlled-release solid oral pharmaceutical formulations of oxcarbazepine which comprises:
a) dissolving oxcarbazepine and mixtures of mono-, di- and triglycerides in a water-miscible organic solvent: ethanol, methanol, dioxane, dimethylformamide and dimethyl sulphoxide;
b) adding an aqueous suspension of water-insoluble excipients to the solution obtained in a) and separating the wet precipitate;
c) adding any water-soluble excipients to the wet precipitate obtained
   in b) and granulating the resulting mixture;
d) compressing or distributing the granulate obtained in c) in the pharmaceutical administration form.

The invention also relates to the oxcarbazepine formulations obtainable by said process.

In another aspect thereof, the invention also relates to oxcarbazepine coacervates obtained by precipitation of oxcarbazepine from a water-miscible solvent with an aqueous suspension of water-insoluble excipients.

### Detailed description of the invention

The purpose of the invention is to produce solid oral controlled (accelerated or delayed) release forms of oxcarbazepine which guarantee the maximum uniformity of dispersion of the drug in suitable matrices, and consequently constant bioavailability and therapeutic efficacy.

The purpose of the invention is achieved by a process which, unlike conventional processes involving grinding/micronisation and mechanical mixing of the active ingredient with the excipients, involves coprecipitation from solutions containing the active ingredient and some excipients dissolved or present in suspension in the solution, as the final step of synthesis of the active ingredient.

Said solution/suspension is added to a solvent in which both the active ingredient and the excipients are insoluble, such as water, under controlled conditions suitable to obtain a microcrystalline coprecipitate or coacervate (with no need for successive micronisations) having a well-defined, uniform, reproducible active ingredient/excipient composition, wherein the active ingredient retains its crystalline type and has a pre-determined particle-size distribution. Said controlled conditions relate to the parameters volume and stirring rate, time and temperature, and are easily controllable and reproducible.

Other insoluble ingredients can be added to the precipitation solvent; they will remain in suspension and will be incorporated uniformly in the precipitate.

Examples of said excipients and ingredients which are insoluble in both the organic solvent and water, the precipitation solvent, for example, are:
Polysaccharides: microcrystalline cellulose and derivatives thereof such as methylcellulose, hydroxypropyl methylcellulose, ethylcellulose, and pineapple fibres.
Inorganic compounds such as talc, colloidal silicon dioxide, calcium phosphate and magnesium phosphate.
Milk protein, soy proteins and gelatin.

Ingredients soluble in the organic solvent and insoluble in water, for example, are mixtures of mono, di- and triglycerides of hydrogenated fatty acids, waxes, alcohols and organic acids with a high molecular weight (> C10).

The precipitate is separated by centrifugation and/or filtration, dried under suitable conditions and granulated.

At this last step, while the product is wet, water-soluble excipients and adjuvants of rapid or delayed release of the active ingredient can be added and amalgamated by mixing. Said adjuvants are dissolved in the imbibition water, and can thus be distributed in a perfectly uniform way. Examples of said excipients are:
Mono-, di- and oligosaccharides: mannitol, xylitol, polyethylene glycols, carboxymethylcellulose sodium, maltodextrins of various molecular weights, guar gum, agar agar and mixtures thereof.
Anionic surfactants such as sodium, potassium or magnesium salts, n-dodecyl sulphates, n-tetradecyl sulphates, n-hexadecyl sulphates, n-octadecyl sulphates, alkylether sulphates such as sodium, potassium or magnesium salts of n-dodecyloxyethyl sulphates, n-tetradecyloxyethyl sulphates, n-hexadecyloxyethyl sulphates and n-octadecyloxyethyl sulphates.

Other non-ionic surfactants are sodium, potassium or magnesium salts of alkanesulphonates: n-dodecanesulphonate, n-tetradecanesulphonate, hexadecanesulphonate, and n-octadecanesulphonate.

Non-ionic surfactants: Esters of fatty acids with polyhydroxylated alcohols such as polyoxyethylene sorbitan monolaurate, monooleate, monostearate, monopalmitate, tristearate and trioleate. Esters of fatty acids with polyoxyethylene glycols 400, 2000. Block copolymers of ethylene/propylene oxide, such as Pluronic and Synperonic.

Polymers such as linear and cross-linked povidone, polyvinyl alcohols.

The end result will be a bulk product ready to be granulated and introduced into sachets or gelatin capsules or, using the direct compression technique, formed into tablets, after the addition of a small amount of lubricants if required.

Its uniform, reproducible composition, particle-size distribution and crystalline type guarantee the constant bioavailability and pharmacological and therapeutic efficacy of the product over time.

Another important factor is that the dilution of the active ingredient in inert excipients may promote the stability of the active ingredient.

The ingredients used will be selected from those able to modulate the immediate or delayed release of the drug, or both actions in two granulates, obtained separately and then mixed in the correct proportions in the pharmaceutical form.

Said proportions between the immediate- and delayed-release forms are preferably between 30 and 50%; the percentage can be chosen on the basis of bioavailability tests.

A scheduled, controlled release is thus effected.

According to the invention, the excipients are selected from the following groups:
- emulsifiers, i.e. adjuvants of the wettability and dispersion in water of the active ingredient;
- inhibitors of the formation of hydrated forms of oxcarbazepine which are difficult to dissolve;
- compressing adjuvants with tablet or granule binding/disintegrating properties;
- binders with properties that delay the release of the active ingredient.

Examples of emulsifiers include:
Anionic surfactants: sodium, potassium or magnesium salts, n-dodecyl sulphates, n-tetradecyl sulphates, n-hexadecyl sulphates, n-octadecyl sulphates, alkylether sulphates such as sodium, potassium or magnesium salts of n-dodecyloxyethyl sulphates, n-tetradecyloxyethyl sulphates, n-hexadecyloxyethyl sulphates, n-octadecyloxyethyl sulphates.

Other surfactants are sodium, potassium or magnesium salts of alkanesulphonates: n-dodecanesulphonate, n-tetradecanesulphonate, hexadecanesulphonate, and n-octadecanesulphonate.

Non-ionic surfactants: Esters of fatty acids with polyhydroxylated alcohols such as polyoxyethylene sorbitan monolaurate, monooleate, monostearate, monopalmitate, tristearate, trioleate, esters of fatty acids with polyoxyethylene glycols 400, 2000, and block copolymers of ethylene/ propylene oxide, such as Pluronic and Synperonic.

Examples of inhibitors of the formation of hydrated forms include block copolymers of ethylene/propylene oxide such as Pluronic and Synperonic, mixtures of mono, di- and triglycerides of hydrogenated fatty acids, waxes, alcohols and organic acids with a high molecular weight (> C10), and polyglycols with a molecular weight of 2000 to 4000.

Examples of compressing adjuvants are mono-, di- and polysaccharides: lactose, saccharose, xylitol, maltose, maltitol, maltodextrins, agar-agar, microcrystalline cellulose and derivatives thereof, such as methylcellulose, hydroxypropyl methylcellulose, ethylcellulose, vegetable fibres, inorganic compounds such as colloidal silicon dioxide, calcium phosphate, magnesium phosphate, proteins of various types such as milk and soy proteins, and vegetable gelatin.

Examples of binders with retardant properties include carboxymethylcellulose sodium, guar gum, agar agar and mixtures thereof.

Mixtures of mono, di- and triglycerides of hydrogenated fatty acids, waxes, alcohols and organic acids with a high molecular weight (> C10), methylcellulose, hydroxypropyl methylcellulose, ethylcellulose, vegetable fibres, and proteins of various types: milk, soya and vegetable gelatin.

According to the invention, all combinations in which the active ingredient is mixed with the other ingredients according to the following principles are possible:
- the oxcarbazepine is dissolved in dimethylformamide or in a solvent miscible with water;
- the components soluble in said solvents but insoluble in water, such as mixtures of mono-, di- and triglycerides, are dissolved together with oxcarbazepine;
- the components insoluble in both organic solvents and water are suspended in water and coprecipitated with oxcarbazepine to obtain a coacervate;
- the water-soluble ingredients are added to the wet precipitate and thoroughly mixed with it so as to dissolve in the water present in the coprecipitate and give a wet granulate, wherein all the ingredients are distributed with the maximum uniformity.

Said uniform distribution of the active ingredient/ingredients guarantees constant bioavailability.

The examples below describe the procedures used, according to the chemico-physical characteristics, to obtain coprecipitates or coacervates between the active ingredient and the other ingredients which modulate the release of the drug in the desired way.

### Example 1

1200 g of oxcarbazepine is dissolved at 60°C under nitrogen in 12 litres of dimethylformamide containing 120 g of a mixture of mono-, di- and triglycerides of vegetable fatty acids having chains of 18 carbon atoms, previously dissolved in dimethylformamide.

The solution is rapidly diluted under energetic stirring in 60 litres of water containing 120 g of microcrystalline cellulose, at a temperature between 0° and 8°C. The resulting precipitate is microcrystalline, with a particle-size distribution range of less than 50 microns, and separated by centrifugation. The yields are practically quantitative.

120 g of mannitol is added to the wet precipitate.

The wet precipitate is then weighed, the residual amount of water is estimated, and the precipitate is distributed on racks for drying under vacuum.

When the moisture has been reduced to approx. 20% of the starting content, the product is granulated to obtain a particle size of between 200 and 300 microns, and dried completely.

The product is then thoroughly mixed with 20 g of talc and 20 g of magnesium stearate and compressed to produce tablets weighing 400 mg with a break line, containing 300 mg of active ingredient. Alternatively, it is compressed to produce oblong tablets weighing 800 mg with a break line, containing 600 mg of active ingredient.

### Example 2

The procedure described in Example 1 is followed, with the variation that 120 g of mannitol is replaced by 120 g of xylitol.

### Example 3

The procedure described in Example 1 is followed, with the variation that 30 g of the 120 g of mannitol is replaced by 30 g of polyethylene glycol 4000.

### Example 4

The procedure described in Example 1 is followed, with the variation that 120 g of mannitol is replaced by 105 g of xylitol and 15 g of carboxymethylcellulose sodium salt.

### Example 5

The procedure described in Example 1 is followed, with the variation that 120 g of mannitol is replaced by 120 g of maltodextrin MW 25,000.

### Example 6

The procedure described in Example 1 is followed, with the variation that the 120 g mixture of mono-, di- and triglycerides consists of vegetable fatty acids having chains with 6 carbon atoms.

### Example 7

The procedure described in Example 1 is followed, with the variation that the 120 g mixture of mono-, di- and triglycerides consists of vegetable fatty acids having chains with 12 carbon atoms.

### Example 8

The procedure described in Example 1 is followed, with the variation that 120 g of mannitol is replaced by 100 g of polyethylene glycol 4000 and 20 g of polyoxyethylene sorbitan monooleate 60.

### Example 9

The procedure described in Example 1 is followed, with the variation that 120 g of mannitol is replaced by 100 g of polyethylene glycol 4000 and 20 g of polyoxyethylene sorbitan monooleate 80.

### Example 10

The procedure described in Example 1 is followed, with the variation that 120 g of mannitol is replaced by 120 g of agar agar.

## Claims

1. A process for the preparation of controlled-release solid oral pharmaceutical formulations of oxcarbazepine which comprises:
a) dissolving the oxcarbazepine and mixtures of mono-, di- and triglycerides in an organic solvent miscible with water;
b) adding an aqueous suspension of water-insoluble excipients to the solution obtained in a) and separating the wet precipitate;
c) adding any water-soluble excipients to the wet precipitate obtained in b) and granulating the resulting mixture;
d) compressing or distributing the granulate obtained in c) in the pharmaceutical administration form.

2. The process as claimed in claim 1, wherein step b) is performed at a temperature of between 0 and 8°C.

3. The process as claimed in claim 2, wherein the microcrystalline co-precipitate obtained has an average particle-size distribution of approx. 50 µm.

4. The process as claimed in any of claims 1-3, wherein the organic solvent miscible with water is dimethylformamide.

5. The process as claimed in any one of claims 1-4, wherein the water-insoluble excipients are selected from microcrystalline cellulose and derivatives thereof, methylcellulose, hydroxypropyl methylcellulose, ethylcellulose, pineapple fibres, talc, colloidal silicon dioxide, calcium phosphate, magnesium phosphate, milk protein, soy proteins and gelatin.

6. The process as claimed in any one of claims 1-5, wherein the water-soluble excipients to be added at step c) are selected from mannitol, xylitol, maltodextrins, sodium carboxymethylcellulose, polyethylene glycols, agar agar and mixtures thereof.

7. Pharmaceutical compositions obtainable by the process as claimed in 1-6.

8. The compositions as claimed in claim 7, in the form of capsules, tablets, and granulates in sachets.

## Patentansprüche

1. Verfahren zur Herstellung von festen oralen pharmazeutischen Formulierungen von Oxcarbazepin mit kontrollierter Freisetzung, das umfasst:
a) Lösen von Oxcarbazepin und Gemischen von Mono-, Di-und Triglyceriden in einem organischen Lösungsmittel, das mit Wasser mischbar ist;
b) Zusetzen einer wässrigen Suspension von wasserunlöslichen Exzipientien zu der in a) erhaltenen Lösung und Abtrennung des nassen Präzipitats;
c) Zusetzen wasserlöslicher Exzipientien zu dem in b) erhaltenen nassen Präzipitat und Granulieren des resultierenden Gemisches;
d) Komprimieren oder Verteilen des in c) erhaltenen Granulats in die pharmazeutische Verabreichungsform.

2. Verfahren gemäß Anspruch 1, wobei Schritt b) bei einer Temperatur von zwischen 0 und 8°C durchgeführt wird.

3. Verfahren gemäß Anspruch 2, wobei das mikrokristalline Copräzipitat, das erhalten wird, eine durchschnittliche Partikelgrößenverteilung von etwa 50 µm hat.

4. Verfahren gemäß einem der Ansprüche 1 bis 3, wobei das organische Lösungsmittel, das mit Wasser mischbar ist, Dimethylformamid ist.

5. Verfahren gemäß einem der Ansprüche 1 bis 4, wobei die wasserunlöslichen Exzipientien aus mikrokristalliner Cellulose und Derivaten davon, Methylcellulose, Hydroxypropylmethylcellulose, Ethylcellulose, Ananasfasern, Talcum, kolloidalem Siliciumdioxid, Calciumphosphat, Magnesiumphosphat, Milchprotein, Sojaproteinen und Gelatine ausgewählt werden.

6. Verfahren gemäß einem der Ansprüche 1 bis 5, wobei die in Schritt c) zuzusetzenden wasserlöslichen Exzipientien aus Mannit, Xylit, Maltodextrinen, Natriumcarboxymethylcellulose, Polyethylenglykolen, Agar-Agar und Gemischen davon ausgewählt werden.

7. Pharmazeutische Zusammensetzungen, die durch das Verfahren, wie es in 1-6 beansprucht ist, erhältlich sind.

8. Zusammensetzungen, wie sie in Anspruch 7 beansprucht sind, in der Form von Kapseln, Tabletten und Granulaten in Sachets.

## Revendications

1. Procédé pour la préparation de formulations pharmaceutiques solides à libération contrôlée d'oxcarbazépine pour une administration par voie orale, qui comprend le fait de :
a) dissoudre l'oxcarbazépine et des mélanges de monoglycérides, de diglycérides et de triglycérides dans un solvant organique miscible à l'eau ;
b) ajouter une suspension aqueuse d'excipients insolubles dans l'eau à la solution obtenue sous a) et séparer le précipité humide ;
c) ajouter tout excipient soluble dans l'eau au précipité humide obtenu sous b) et granuler le mélange résultant ;
d) comprimer ou distribuer le produit de granulation obtenu sous c) dans la forme d'administration pharmaceutique.

2. Procédé selon la revendication 1, dans lequel l'étape b) est mise en oeuvre à une température entre 0 et 8 °C.

3. Procédé selon la revendication 2, dans lequel le coprécipité microcristallin que l'on obtient possède une distribution granulométrique moyenne d'approximativement 50 µm.

4. Procédé selon l'une quelconque des revendications 1 à 3, dans lequel le solvant organique miscible à l'eau est le diméthylformamide.

5. Procédé selon l'une quelconque des revendications 1 à 4, dans lequel les excipients insolubles dans l'eau sont choisis parmi la cellulose microcristalline et ses dérivés, la méthylcellulose, l'hydroxypropylméthylcellulose, l'éthylcellulose, des fibres d'ananas, du talc, du dioxyde de silicium colloïdal, du phosphate de calcium, du phosphate de magnésium, de la protéine de lait, des protéines de soja et la gélatine.

6. Procédé selon l'une quelconque des revendications 1 à 5, dans lequel les excipients solubles dans l'eau à ajouter à l'étape c) sont choisis parmi le mannitol, le xylitol, les maltodextrines, la carboxyméthylcellulose de sodium, des polyéthylèneglycols, l'agar-agar et leurs mélanges.

7. Compositions pharmaceutiques que l'on peut obtenir via le procédé selon les revendications 1 à 6.

8. Compositions selon la revendication 7 sous la forme de capsules, de comprimés et de granulés dans des sachets.
